(19) Europäisches Patentamt
European Patent Office
Office européen des brevets

(11) **EP 1 380 302 B1**

(12) **EUROPEAN PATENT SPECIFICATION**

(45) Date of publication and mention
of the grant of the patent:
**20.08.2008 Bulletin 2008/34**

(51) Int Cl.:
*A61K 31/57* (2006.01)    *A61P 27/02* (2006.01)

(21) Application number: **02708676.8**

(22) Date of filing: **27.03.2002**

(86) International application number:
**PCT/JP2002/002950**

(87) International publication number:
**WO 2002/078713 (10.10.2002 Gazette 2002/41)**

(54) **REMEDIES FOR RETINA AND CHOROID DISEASES CONTAINING STEROIDS AS THE ACTIVE INGREDIENT**

HEILMITTEL FÜR ERKRANKUNGEN DER RETINA UND DER ADERHAUT DIE ALS AKTIVE BESTANDTEILE STEROIDE ENTHALTEN

MEDICAMENTS CONTRE LES AFFECTIONS RETINIENNES ET CHOROIDIENNES CONTENANT DES STEROIDES COMME PRINCIPE ACTIF

(84) Designated Contracting States:
**AT BE CH CY DE DK ES FI FR GB GR IE IT LI LU MC NL PT SE TR**

(30) Priority: **28.03.2001 JP 2001092329**

(43) Date of publication of application:
**14.01.2004 Bulletin 2004/03**

(73) Proprietor: **SANTEN PHARMACEUTICAL CO., LTD.**
**Higashiyodogawa-ku,**
**Osaka-shi,**
**Osaka 533-8651 (JP)**

(72) Inventors:
• **TANO, Yasuo**
  **Kobe-shi,**
  **Hyogo 658-0064 (JP)**
• **KUROSE, Tatsuji,**
  **c/o SANTEN PHARMACEUTICAL CO. LTD**
  **Ikoma-shi,**
  **Nara 630-0101 (JP)**

(74) Representative: **Peaucelle, Chantal et al**
**Cabinet Armengaud Aîné**
**3, Avenue Bugeaud**
**75116 Paris (FR)**

(56) References cited:

EP-A1- 0 488 401          WO-A1-00/56340
WO-A1-91/03245            WO-A1-93/12808
WO-A1-97/05895            WO-A1-98/47366
WO-A2-02/02076            WO-A2-93/10141
WO-A2-98/19649            JP-A- 61 106 521
US-A- 5 104 787

• IKEDA TSUNEHIKO; DANJO SHINJI; TANO YASUO: "Value of pupillary stretching for small fixed pupil vitreoretinal surgery" JAPANESE JOURNAL OF CLINICAL OPHTHALMOLOGY, vol. 47, no. 7, 1993, pages 1379-1383, XP009061620
• HAYASHI M; TAJIKA H; NAITO T; SHIOTA H: "A case of proliferative diabetic retinopathy with increasing blood glucose level after subconjunctival corticosteroid injection" FOLIA OPHTHALMOLOGICA JAPONICA, vol. 52, no. 2, 2001, pages 147-149, XP009061619
• CEKIC O ET AL: "Effect of capsulorhexis size onpostoperative intraocular pressure" JOURNAL CATARACT AND REFRACTIVE SURGERY, AMERICAN SOCIETY OF CATARACT AND REFRACTIVE, US, vol. 25, no. 3, March 1999 (1999-03), pages 416-419, XP004738812 ISSN: 0886-3350
• SIMON ET AL: "Corneal Edema After Pediatric Cataract Surgery" JOURNAL OF THE AAPOS, vol. 1, no. 2, 1997, pages 102-104, XP002368147
• KOIZUMI, K. AND HONDA, Y. NIPPON GANKA KIYO vol. 34, no. 7, 1983, pages 1484 - 1487, XP002953141
• HARA, S. NIPPON GANKA GAKKAI ZASSHI vol. 81, no. 7, 1977, pages 720 - 727, XP002953142

**Description**

Technical Field

The present invention relates to therapeutic agents for retinochoroidal disorders comprising a specific steroid, namely betamethasone or hydrocortisone as an active ingredient and to subconjunctival injections for treating the retinochoroidal disorders comprising the steroid as an active ingredient.

Background Art

Retinochoroidal disorders are intractable disorders leading to blindness. In particular, age-related macular degeneration, diabetic retinopathy and proliferative vitreoretinopathy are main retinochoroidal disorders.

Age-related macular degeneration (hereinafter abbreviated as "AMD") is a disorder which is caused by an unknown origin and occurs at a macular site with aging. This disorder often occurs among old people over fifty and is noted as a disorder which is a main cause of visual loss and blindness of old people in recent years. AMD is classified into two types, i.e., an exudation-type wherein neovascularization derived from choroid extends to the macular site to cause hemorrhage or exudation, and an atrophy-type in which choroidal neovascularization does not participate and wherein retinal pigment epithelial cells and a choroidal capillary plate atrophy. In the exudation-type, neovascularization extends or migrates from the choroids to the retinal pigment epithelial cells or under the retina in macula lutea of old people to cause hemorrhage or exudative lesions.

Diabetic retinopathy (hereinafter abbreviated as "DR") is an ophthalmic complication of diabetes and is a disorder which ranks first in causes of blindness among adults in recent years. Since aging of population progresses and patients suffering from diabetes live longer, frequency of DR pathogenesis is increasing. DR is a disease of the retinal vessels and progresses as angiopathy at a capillary level. An initial lesion of angiopathy is called simple retinopathy, a state where the lesion progresses and obstruction of capillary progresses is called preproliferative retinopathy, and a state where the obstruction of angiopathy is extended, retinal ischemia progresses, and neovascularization is caused in a vitreoretinal body is called proliferative retinopathy.

Proliferative vitreoretinopathy (hereinafter abbreviated as "PVR") is a serious complication on which rhegmatogenous retinal detachment supervenes. Though frequency of proliferative vitreoretinopathy is said to be 5 to 10% of retinal detachment, the frequency tends to increase in recent years as vitreous surgery becomes popular. Essence of this pathema consists in proliferation of cells which do not originate from blood vessels such as retinal pigment epithelial cells, retinal glia cells and fibroblasts. The proliferated substance of retina is formed on a front or rear of the detached retina or in a vitreous body, and the retina is strongly pulled to cause total detachment.

The retinochoroidal disorders are treated mainly by surgical operations. Though treatment of disorders of an outer ocular area is mainly pharmacotherapy by instillation or the like, drugs are hardly delivered to the retina, which makes pharmacotherapy of the retinochoroidal disorders difficult. Though treatment of the retinochoroidal disorders by an intravenous injection or oral administration is also attempted, systemic actions of the drugs appear remarkably. Accordingly, a method of injecting drugs directly into the vitreous body are studied.

It is already known that steroids are useful as the drugs to be used for treatment of the retinochoroidal disorders, and particularly triamcinolone is reported variously. For example, U.S. Pat. No. 5,770,589 discloses a method of treating macular degeneration by injecting triamcinolone into a vitreous body. Am. J. Ophthal. 89:131-136, 1980 reports that proliferation of fibroblasts transplanted into a vitreous body is inhibited by injecting triamcinolone acetonide into the vitreous body, which is useful for treatment of PVR. J. Ocul. Pharmacol.Ther. 15 (5), 425-428, 1999 reports that a sub-Tenon's injection of triamcinolone exhibits an effect on inhibition of choroidal neovascularization.

However, it is unknown that betamethasone and hydrocortisone are used for treatment of the retinochoroidal disorders among the steroids. Though the intravitreous injection and the sub-Tenon's injection are known as methods of administering the steroids, there has been no report concerning utility of a subconjunctival injection in a method of administration in treatment of the retinochoroidal disorders.

Though it is known that triamcinolone is useful for treatment of the retinochoroidal disorders as mentioned above, triamcinolone is not satisfactory yet in terms of pharmaceutical effects. It is a very interesting subject to find steroids which exhibit higher effects.

Even if it is known that the steroids are useful for the retinochoroidal disorders, it is an important subject how the steroids are delivered to retinochoroid efficiently. Though the intravitreous injection and the sub-Tenon's injection are proposed as delivery means, these depend greatly on skill of doctors. Further, since disorders of ophthalmic tissues are serious, patients suffer considerable pain or the like. Accordingly, it is desired to develop therapeutic agents wherein a drug delivery to the retina, convenience of administration, and the disorders of the ophthalmic tissues are well-balanced.

In JAPANESE JOURNAL OF CLINICAL OPHTHALMOLOGY, vol. 47, no. 7, 1993, pages 1379-1383, "Value of pupillary stretching for small fixed pupil vitreoretinal surgery, IKEDA TSUNEHIKO et al. describe that subconjunctival injection of steroid is useful for fibrin deposition which is a complication associated with the pupillary stretching surgery.

In FOLIA OPHTHALMOLOGICA JAPONICA, vol. 52, no. 2, 2001, pages 147-149, "A case of proliferative diabetic retinopathy with increasing blood glucose level after subconjunctival corticosteroid injection", HAYASHI M et *al.* disclose that subconjunctival dexamethasone injection is useful for anti-inflammation in anterior chamber relative to fibrin deposition after the vitreous body surgery.

WO 00/56340-A1 describes a method for treating diabetic retinopathy by inhibiting the activity of vascular permeability factor with use of SP-PG.

EP-A1-0 488 401 describes sustained-release pharmaceutical preparation (preparation for intra-ocular implant) to be applied to the interior of the eye after a surgical operation.

Disclosure of the Invention

Studying precisely, the present inventors found that betamethasone and hydrocortisone exhibit excellent inhibitory actions on choroidal neovascularization, inhibitory actions on hyperpermeability of vessels and inhibitory actions on retinal detachment, and these compounds are useful as therapeutic agents for retinochoroidal disorders, particularly age-related macular degeneration, diabetic retinopathy and proliferative vitreoretinopathy. It was also found that by injecting the steroids subconjunctivally, they can be administered more easily and can be delivered to retinochoroid efficiently. A known intravitreous injection is a method of injecting a drug directly into a vitreous body close to retinal tissues, and a sub-Tenon's injection is a method of injecting the drug into a Tenon's sac, which is a fine tissue. Since these are administration to sites which cannot be seen directly, they need advanced techniques, and a burden on patients is heavy, consequently administration times are restricted. Further, since an injection needle reaches an intraocular site, intraocular infection may be induced. On the other hand, since the subconjunctival injection is an injection into sites which can be seen directly, manoeuvre is relatively easy, burden on patients is light, and administration times are not so restricted.

A first invention is characterized by the kind of drug, namely steroid. This invention relates to therapeutic agents for the retinochoroidal disorders comprising betamethasone or hydrocortisone (these steroid compounds are hereinafter referred to as "the present compounds") as an active ingredient. They can be in the form of esters or salts. The esters in the present compounds can be any pharmaceutically acceptable esters and are exemplified by phosphates, maleates, acetates, formates and the like.

The salts can be any pharmaceutically acceptable salts and are exemplified by sodium salts, potassium salts and the like. Preferred examples of the present compounds are betamethasone sodium phosphate and hydrocortisone.

In order to study utility of the present compounds for treatment of the retinochoroidal disorders, the inhibitory actions on choroidal neovascularization, the inhibitory actions on hyperpermeability of vessels and the inhibitory actions on retinal detachment were studied. Details will be described in Examples below.

Though a preferred administration form of the present compounds is the subconjunctival injection, a usual administration form of steroids can also be used depending on skill of doctors, symptoms of patients and the like. Namely, in the first invention, betamethasone or hydrocortisone can be administered parenterally or orally. Examples of parenteral dosage forms are injections and ophthalmic solutions, examples of oral dosage forms are tablets, capsules, granules and powder, and the present compounds can be formulated into preparations by the conventional methods. For example, the injections can be prepared by adding widely-used additives such as an osmotic pressure adjustor such as sodium chloride, a pH adjustor such as sodium phosphate, a surfactant such as polysorbate 80 or a thickener such as methyl cellulose to the present compound and by dissolving the mixture in distilled water for injection. These injections can be used not only as the subconjunctival injection but also as the intravitreous injection and the sub-Tenon's injection.

The dosage of the present compound can be adjusted depending on symptoms, age and the like. In the case of the injections, the usual dose can be 1 $\mu$g to 10 mg, preferably 10 $\mu$g to 1 mg a time.

A second invention relates to therapeutic agents for the retinochoroidal disorders by injecting the steroids such as the above-mentioned present compounds subconjunctivally. This invention is characterized by an administration site and is not limited by the kind of steroid to be used. Namely, in the second invention steroids can be any kinds which are useful for the retinochoroidal disorders such as triamcinolone in addition to the present compounds. The results of pharmacological tests below show explicitly effects of the second invention, namely the steroids reach a retinochoroidal disorder site and can exhibit their drug efficacy by the subconjunctival injection which is useful for both doctors and patients. A process for preparation, dosage and the like of injections to be used in the second invention are the same as those of the first invention.

Formulation Example and results of pharmacological tests will be shown below.

The present invention includes a method of treating the retinochoroidal disorders comprising administering to patients an effective amount of betamethasone or hydrocortisone which can be in the form of the esters or the salts.

The present invention also includes the use of betamethasone, an ester or a salt thereof for treating the retinochoroidal disorders comprising administering subconjunctivally to patients an effective amount of the steroid.

Best Mode for Carrying out the Invention

Formulation Example

A general formulation example of an injection of the present compound is shown below.

1) Injection

|  |  |
|---|---|
| In formulation 100 ml |  |
| Betamethasone phosphate | 20 mg |
| Sodium chloride | 900 mg |
| Distilled water for injection | quantum sufficit. |

Pharmacological tests

In order to study effects of the present compounds on AMD and DR, choroidal neovascularization inhibition tests and retinal vessel permeability inhibition tests were performed. In order to study effects on PVR, retinal detachment inhibition tests were performed.

1. Choroidal neovascularization inhibition tests of betamethasone sodium phosphate

Preparation of laser-induced rat choroidal neovascularization models

A one ml/kg mixed solution (7:1) of a 5% ketamine hydrochloride injection and a 2% xylazine hydrochloride injection was administered intramuscularly to rats to anesthetize them systemically. A 0.5% tropicamide-0.5% phenylephrine hydrochloride ophthalmic solution was instilled into the eyes to cause mydriasis, and then photocoagulation was performed with a krypton laser photocoagulation apparatus. The photocoagulation was carried out in a posterior section of ocular fundus at eight spots per eye sparsely avoiding thick retinal vessels and focusing on the retinal depth (coagulation conditions: spot size: 100 $\mu$m, output: 100 mW, coagulation time: 0.1 sec). After the photocoagulation, the ocular fundus was photographed to confirm laser irradiation sites.

Method of administering drug

Betamethasone sodium phosphate was dissolved in phosphate buffers so that its concentrations were 0.2 mg/ml and 2 mg/ml, and each solution was administered subconjunctivally in an amount of 50 $\mu$l for seven days after the laser irradiation. Triamcinolone was suspended in the phosphate buffers as a comparative group so that its concentrations were 0.2 mg/ml and 2 mg/ml, and each suspension was administered similarly. The phosphate buffer was administered similarly as a vehicle administration group.

Method of evaluation

On seventh day after the photocoagulation, 0.1 ml of 10% fluorescein was injected from carotid vein, and fluorescein fundus angiography was performed. In the fluorescein fundus angiography, a spot where fluorescence diapedesis was not observed was judged as negative, and a spot where fluorescence diapedesis was observed was judged as positive. Each neovascularization exhibition rate was obtained by calculating the ratio of a positive spot number to eight spots irradiated with the laser, and each choroidal neovascularization inhibition rate was calculated according to the following equation. Choroidal neovascularization inhibition rate (%) = $(A_0-A_X)/A_0 \times 100$ $A_0$: Neovascularization exhibition rate of vehicle administration group $A_X$: Neovascularization exhibition rate of drug administration group Results
Results of the above-mentioned tests are shown in Table 1. It was found that betamethasone sodium phosphate exhibits choroidal neovascularization inhibition rates which are higher than those of triamcinolone at both dosages of 10 $\mu$g/eye (one eye)/day (one day) and 100 $\mu$g/eye/day, and betamethasone sodium phosphate has an inhibitory effect which is about three times stronger than that of triamcinolone at 10 $\mu$g/eye/day.

Table 1

| | Neovascularization exhibition rate (%, average of eight eyes) | Choroidal neovascularization inhibition rate (%) |
|---|---|---|
| Vehicle administration group | 54.2 | - |
| Betamethasone sodium phosphate administration group 10 µg/eye/day | 34.4 | 36.3 |
| 100 µg/eye/day | 11.1 | 79.4 |
| Triamcinolone administration group 10 µg/eye/day | 46.9 | 13.1 |
| 100 µg/eye/day | 22.3 | 58.8 |

2. Choroidal neovascularization inhibition tests of hydrocortisone Preparation of laser-induced rat choroidal neovascularization models

Laser-induced rat choroidal neovascularization models were prepared by a method similar to the above-mentioned tests of betamethasone sodium phosphate.

Method of administering drug

Hydrocortisone was dissolved in phosphate buffers so that its concentrations were 2 mg/ml and 20 mg/ml, and each solution was administered once subconjunctivally in an amount of 50 µl immediately after laser irradiation. The phosphate buffer was administered similarly as a vehicle administration group.

Method of evaluation

Evaluation was carried out by a method similar to the above-mentioned tests of betamethasone sodium phosphate.

Results

Results of the above-mentioned tests are shown in Table 2. It was found that hydrocortisone has an inhibitory effect on choroidal neovascularization at both dosages of 100 µg/eye/day and 1,000 µg/eye/day.

Table 2

| | Neovascularizat ion exhibition rate (%, average of eight eyes) | Choroidal neovasculari zation inhibition rate (%) |
|---|---|---|
| Vehicle administration group | 67.2 | - |
| Hydrocortisone administration group 100 µg/eye/day | 45.3 | 32.6 |
| 1000 µg/eye/day | 19.6 | 70.8 |

3. Retinal vessel permeability inhibition tests of betamethasone phosphate

Preparation of thrombin-induced rat retinal vessel hyperpermeability models

A one ml/kg mixed solution (7:1) of a 5% ketamine hydrochloride injection and a 2% xylazine hydrochloride injection was intramuscularly administered to rats to anesthetize them systemically. Then, a 0.5% tropicamide-0.5% phenylephrine hydrochloride ophthalmic solution was instilled into left eyes to cause mydriasis. Thrombin (500 U/ml, 2.5 µl) was injected into vitreous bodies (left eye) of rats of a drug administration group and a control group with a 33G needle without injuring crystalline lenses and retinas while observing them with a microscope for operation. A vehicle (Dulbecco's modified phosphate buffer physiological saline) was administered to rats of a normal group instead of thrombin.

Method of administering drug

Betamethasone sodium phosphate was dissolved in physiological saline so that its concentration was 2 mg/ml or 20 mg/ml. A 0.4% oxybuprocaine ophthalmic solution was instilled into the left eyes of the rats one hour before and 23 hours after administering thrombin or the vehicle (Dulbecco's modified phosphate buffer physiological saline), and then 2 mg/ml or 20 mg/ml betamethasone phosphate was injected subconjunctivally into the rats of the drug administration group. The physiological saline was injected subconjunctivally into the rats of the normal group and the control group.

Method of evaluation

Forty-eight hours after administering thrombin intravitreously, the 0.5% tropicamide-0.5% phenylephrine hydrochloride ophthalmic solution was instilled into the eyes to cause mydriasis. The one ml/kg mixed solution (7:1) of the 5% ketamine hydrochloride injection and the 2% xylazine hydrochloride injection was intramuscularly administered to rats to anesthetize them systemically, and then a 1% fluorescein solution (1 ml/kg) was administered intravenously to penes. About 40 minutes after administering fluorescein, blood was collected from hearts, and then an intraocular fluorescent pigment concentration was immediately measured with a fluorotron master. The collected blood was centrifuged at 15,000 rpm $\times$ 5 minutes, then supernatant (plasma) was diluted 51 times with the phosphate buffer physiological saline, and a fluorescent pigment concentration in the plasma was measured with the fluorotron master. About 45 minutes after administering fluorescein obtained with the fluorotron master, the intraocular fluorescent pigment concentration was divided by the fluorescent pigment concentration in the plasma to obtain retinal vessel permeability. Results
Results are shown in Table 3. Comparison between the normal group and the control group shows that hyperpermeability of retinal vessels due to thrombin was observed in the control group. However, it was shown that when betamethasone sodium phosphate is administered with thrombin, the hyperpermeability of retinal vessels due to thrombin is inhibited.

Table 3

|  | Retinal vessel permeability |
| --- | --- |
| Normal group (vehicle + saline administration) | 1.72 |
| Control group (thrombin + saline administration) | 3.31 |
| Thrombin + betamethasone sodium phosphate administration group (2 mg/ml) | 1.85 |
| Thrombin + betamethasone sodium phosphate administration group (20 mg/ml) | 1.54 |

4. Retinal detachment inhibition tests of betamethasone

Preparation of dispase-induced rabbit PVR models

A 0.5% tropicamide-0.5% phenylephrine hydrochloride ophthalmic solution was instilled into eyes of rabbits to cause mydriasis, and a 1 ml/kg mixed solution (7:1) of a 5% ketamine hydrochloride injection and a 2% xylazine hydrochloride injection was administered intramuscularly to the rabbits to anesthetize them systemically. 0.4% oxybuprocaine was instilled into the eyes to anesthetize anterior segments of the eyes, and then 100 $\mu$l of a dispase solution prepared at a concentration of 0.05 U/100 $\mu$l with physiological saline was injected into vitreous bodies. Six weeks and ten weeks after injecting the dispase solution, PVR induced by dispase was confirmed by observing ocular fundus.

Administration of drug

Betamethasone was dissolved in a phosphate buffer so that its concentration was 0.1 g/ml, and the solution was administered subconjunctivally in an amount of 100 $\mu$l immediately after injecting dispase. The phosphate buffer was administered similarly as a vehicle administration group.

Method of evaluation

The ocular fundus was observed 56 days after inducing by dispase, and PVR was judged by scores. Criteria of judgment are shown below. Scores of three or more were defined as exhibition of PVR, and pathogenesis rates of PVR (pathogenesis rates of retinal detachment) in respective groups were calculated according to the equation 2.

0: Normal retina
1: A vitreous membrane is observed.
2: Traction, vascular abnormality or engorgement of the retina is observed.
3: Topical retinal detachment is observed.
4: Wide retinal detachment is observed.
5: Total retinal detachment is observed.

$$\text{Pathogenesis rate of PVR (\%)} = \text{PVR exhibition eye number} \diagup \text{test eye}$$

$$\text{number} \times 100$$

Results

Results are shown in Table 4. Table 4 shows that pathogenesis rates of PVR in the vehicle administration group and the betamethasone administration group are 66.7% and 37.5% respectively, and it was found that betamethasone inhibits PVR.

Table 4

|  | After 6 weeks | After 10 weeks |
|---|---|---|
| Vehicle administration group | 66.7% | 66.7% |
| Betamethasone administration group 10 mg/eye | 45.5% | 37.5% |

Industrial Applicability

Since betamethasone and hydrocortisone of the present invention have excellent inhibitory actions on choroidal neovascularization, inhibitory actions on hyperpermeability of vessels and inhibitory actions on PVR, they are useful as therapeutic agents for retinochoroidal disorders such as therapeutic agents for age-related macular degeneration and therapeutic agents for proliferative vitreoretinopathy. When injections comprising the steroid of the present invention are injected subconjunctivally, the retinochoroidal disorders can be easily treated.

**Claims**

1. Use of betamethasone which can be in the form of an ester or a salt in the manufacture of a therapeutic agent for a retinochoroidal disorder, wherein the retinochoroidal disorder is age-related macular degeneration or diabetic retinopathy.

2. The use as claimed in claim 1, wherein the dosage form of the therapeutic agent is an injection.

3. The use as claimed in claim 2, wherein the dosage of betamethasone is 1 µg to 10 mg.

4. The use as claimed in any one of claims 1 to 3, wherein the therapeutic agent is administered subconjunctivally or by a sub-Tenon's injection.

**Patentansprüche**

1. Verwendung von Betamethason, das in Form eines Esters oder eines Salzes vorliegen kann, zur Herstellung eines therapeutischen Mittels gegen eine retinochoroidale Störung, wobei es sich bei der retinochoroidalen Störung um altersbedingte Makuladegeneration oder diabetische Retinopathie handelt.

2. Verwendung gemäß Anspruch 1, wobei die Darreichungsform des therapeutischen Mittels eine Injektion ist.

**3.** Verwendung gemäß Anspruch 2, wobei die Dosis von Betamethason 1 $\mu$g bis 10 mg beträgt.

**4.** Verwendung gemäß einem der Ansprüche 1 bis 3, wobei das therapeutische Mittel subkonjunktival oder durch eine Sub-Tenon-Injektion verabreicht wird.

**Revendications**

**1.** Utilisation de bétaméthasone qui peut être sous la forme d'un ester ou d'un sel dans la fabrication d'un agent thérapeutique pour un trouble rétino-choroïdien, dans laquelle le trouble rétino-choroïdien est une dégénérescence maculaire liée à l'âge ou une rétinopathie diabétique.

**2.** Utilisation selon la revendication 1, dans laquelle la forme galénique de l'agent thérapeutique est une injection.

**3.** Utilisation selon la revendication 2, dans laquelle la dose de bétaméthasone va de 1 $\mu$g à 10 mg.

**4.** Utilisation selon l'une quelconque des revendications 1 à 3, dans laquelle l'agent thérapeutique est administré de manière sous-conjonctivale ou par une injection sous-ténonienne.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5770589 A
- WO 0056340 A1
- EP 0488401 A1

**Non-patent literature cited in the description**

- *Am. J. Ophthal.,* 1980, vol. 89, 131-136
- *J. Ocul. Pharmacol.Ther.,* 1999, vol. 15 (5), 425-428
- **IKEDA TSUNEHIKO.** Value of pupillary stretching for small fixed pupil vitreoretinal surgery. *JAPANESE JOURNAL OF CLINICAL OPHTHALMOLOGY,* 1993, vol. 47 (7), 1379-1383
- **HAYASHI M.** A case of proliferative diabetic retinopathy with increasing blood glucose level after subconjunctival corticosteroid injection. *FOLIA OPHTHALMOLOGICA JAPONICA,* 2001, vol. 52 (2), 147-149